# EUROPÄISCHE PATENTANMELDUNG

(11) **EP 0 597 220 A2**
(43) Veröffentlichungstag der Anmeldung: **18.05.1994**
(21) Anmeldenummer: 93115321.7
(22) Anmeldetag: 23.09.1993
(51) Int. Cl.: A61M 5/168, A61M 5/162, A61M 5/50

(54) **Medizinisches Übertragungsgerät**

(30) Priorität: 06.11.1992 DE 4237436
(71) Anmelder: B. BRAUN MELSUNGEN AG, D-34212 Melsungen (DE)
(72) Erfinder: Herold, Manfred, Dr., D-34212 Melsungen (DE); Brethauer, Ulrich, D-34327 Körle (DE); Fuchs, Jürgen, D-34308 Emstal (DE)
(74) Vertreter: Selting, Günther, Dipl.-Ing.

(57) **Zusammenfassung**

Bei einem medizinischen Übertragungsgerät besteht die Schutzkappe (11) aus einem abnehmbaren Kappenkörper (17) und einem unlösbar mit dem medizinischen Gerät (10) verbundenen Halteteil (18). Kappenkörper und Halteteil sind durch mindestens ein Abreißteil (19) miteinander verbunden. Beim Abnehmen des Kappenkörpers (17) reißen die Abreißteile (19), so daß das Halteteil (18) am Gerät (10) verbleibt und anzeigt, daß ein Dekonnektierung des Kappenkörpers erfolgt ist. Dadurch wird eine Originalitätsgarantie bewirkt. Der Kappenkörper (17) umschließt mit einem Sitz (20) den Ansatz (15) des Gerätes (10) keimdicht. Dadurch im Originalzustand die Sterilhaltung des von der Schutzkappe (11) umschlossenen Schafts (16) sichergestellt. Die nicht abgebrochene Schitzkappe liefert eine Garantie für die Innensterilität des Gerätes.

## Beschreibung

Die Erfindung betrifft ein medizinisches Übertragungsgerät mit einem Schlauch, an dessen einem Ende eine Tropfkammer und an dessen anderem Ende ein Anschlußstück vorgesehen ist.

Medinizinische Übertragungsgeräte, die zum einmaligen Gebrauch hergestellt werden, sind an dem Einstechdorn, der durch einen Elastomerstopfen einer Falsche hindurchgestochen wird, mit einer Schutzkappe aus Kunststoff ausgerüstet. Die wesentliche Funktion der Schutzkappe ist der Schutz des Anwenders vor Verletzungsgefahr, die Verhinderung der Beschädigung der Sterilverpakkung und die Vermeidung von Verschmutzung oder Beschädigung des Einstechdornes. Üblicherweise ist die Schutzkappe auf den Ansatz des Einstechdorns reibschlüssig aufgesteckt, so daß sie unter Transportbedingungen nicht abfallen kann, andererseits aber von dem Anwender leicht abgenommen werden kann.

Aus AT-B-394 950 ist eine Injektionsspritze mit Nadelschutzkappe bekannt, bei der die Nadelschutzkappe einstückig mit einem Basisteil ausgebildet ist, das über dem Spritzenabsatz sitzt. Das Basisteil ist über eine Sollbruchstelle mit einem trennbaren Kappenteil verbunden, welches die Kanüle umgibt. Zur Benutzung der Injektionsspritze muß das trennbare Kappenteil abgenommen werden, wobei es an der Sollbruchstelle von dem Basisteil abreißt. Durch die zweiteilige Ausbildung der Schutzkappe wird ein Originalitätsverschluß erhalten, so daß anhand des Zerbrechens der Sollbruchstelle die Öffnung der sterilen Verpackung erkannt werden kann. Das trennbare Kappenteil bewirkt ferner beim Losdrehen das Drehen eines Gewindeteils, welches die als Doppelkanüle ausgebildete Injektionsnadel vorschiebt, wodurch die Dichtscheibe einer Spritze durchstochen wird. Der Hals der Spritze wird von dem Basisteil der Verschlußkappe ebenfalls umfaßt und festgehalten. Dieses Prinzip ist bei Übertragungsgeräten zum Übertragen einer Flüssigkeit aus einer Flasche nicht geeignet, weil die Flasche nicht Bestandteil der Übertragungsvorrichtung ist. Übertragungsvorrichtungen sollen vielmehr an eine Flasche außen angeschlossen werden, die aus einer Vielzahl von Flaschen mit unterschiedlichen Inhalten auswählbar ist.

Der Erfindung liegt die Aufgabe zugrunde, ein medizinisches Übertragungsgerät mit Tropfkammer, Schlauch und Anschlußstück zur Verfügung zu stellen, das eine Garantiefunktion im Sinne eines Originalitätsverschlusses bietet.

Die Lösung dieser Aufgabe erfolgt erfindungsgemäß mit den im Patentanspruch 1 angegebenen Merkmalen.

Bei dem erfindungsgemäßen Übertragungsgerät besteht die Schutzkappe des Einstechdornes und/oder des Anschlußstücks aus einem abtrennbaren Kappenkörper und einem unlösbar mit dem medizinischen Gerät verbundenen Halteteil. Kappenkörper und Halteteil sind durch mindestens ein Abreißteil miteinander verbunden. Das Halteteil wird durch Verpressung, Verschnappung, Anpunkten oder durch Schweißung oder Verklebung irreversibel und nichtlösbar an dem medizinischen Gerät gehalten. Beim Abnehmen des Kappenkörpers reißt das mindestens eine Abreißteil, so daß der Kappenkörper von dem Halteteil abgetrennt wird, das am Gerät verbleibt und damit anzeigt, daß eine Dekonnektierung des Kappenkörpers erfolgt ist. Hierdurch wird eine Originalitätsgarantie bewirkt, indem das erstmalige Dekonnektieren des Kappenkörpers erkennbar ist. Wird beim Abnehmen des Kappenkörpers festgestellt, daß das Abreißteil bereits zerbrochen ist, dann wird erkannt, daß das Gerät nicht mehr im Originalitätszustand ist.

Wenn die Verbindung Kappenkörper/Kappensitz keimdicht ist, hat die Schutzkappe die weitere Funktion, daß sie im Originalitätszustand die Sterilhaltung des vom Kappenkörper abgedeckten Teil sowie des Innenlumens des Gesamtgerätes sicherstellt. Damit kann in zahlreichen Fällen auf eine sterile Einzelverpackung des medizinischen Gerätes verzichtet werden. Die nicht abgebrochene Schutzkappe liefert eine Garantie für die Innensterilität des Übertragungsgerätes, d.h. für die Sterilität des Teils, der mit dem Patienten oder mit anderen sterilen Einrichtungen in Verbindung kommt. Eine Außensterilität des Gerätes ist in vielen Fällen nicht erforderlich. Auf diese Weise kann auf die Einzelverpackung des Gerätes verzichtet werden, wodurch sich der Verpackungsaufwand verringert und weniger Verpackungsmüll im Krankenhaus entsteht.

Vorzugsweise umschließt der Kappenkörper keimdicht einen Sitz am medizinischen Übertragungsgerät, um die Innensterilität des Gerätes zu gewährleisten. Durch den Eingriff des Kappenkörpers mit dem Sitz entsteht ein geschlossener Hohlraum, der das zu schützende Teil umschließt bzw. das Gesamtgerät abschließt. Bei Gamma-sterilisation erhält man aufgrund des Durchdringungsvermögens der Strahlen trotz aufgesetzter, hermetisch abdichtender Schutzkappe die erwünschte Innensterilität des medizinischen Gerätes. Bei Gas- oder Dampfsterilisation kann die Schutzkappe mit einem gasdurchlässigen Sterilfilter ausgestattet sein.

Vorzugsweise erfolgt das Abtrennen des Kappenkörpers von dem Halteteil durch Drehen des Kappenkörpers und durch Abscheren des mindestens einen Abreißteils. Die hierfür erforderliche Abscherkraft kann relativ leicht mit der Hand aufgebracht werden. Voraussetzung ist, daß die Schutzkappe derart gestaltet ist, daß sie abrutschsicher leicht gedreht werden kann. Hierfür kann die Schutzkappe z.B. mit einer längslaufenden Profilierung, mit abstehenden Flügeln u. dgl. versehen sein.

Im folgenden werden unter Bezugnahme auf die Zeichnungen Ausführungsbeispiele der Erfindung näher erläutert.

Es zeigen:
Fig. 1 eine schematische Darstellung eines mit der Schutzkappe versehenen medizinischen Übertragungsgerätes,
Fig. 2 eine vergrößerte Darstellung eines Teils des Gerätes mit der einen Schutzkappe, teilweise geschnitten,
Fig. 3 einen Längsschnitt durch eine andere Ausführung der Schutzkappe,
Fig. 4 eine weitere Ausführungsform der Schutzkappe und
Fig. 5 einen Schnitt durch ein anderes Ausführungsbeispiel des Anschlußstücks.

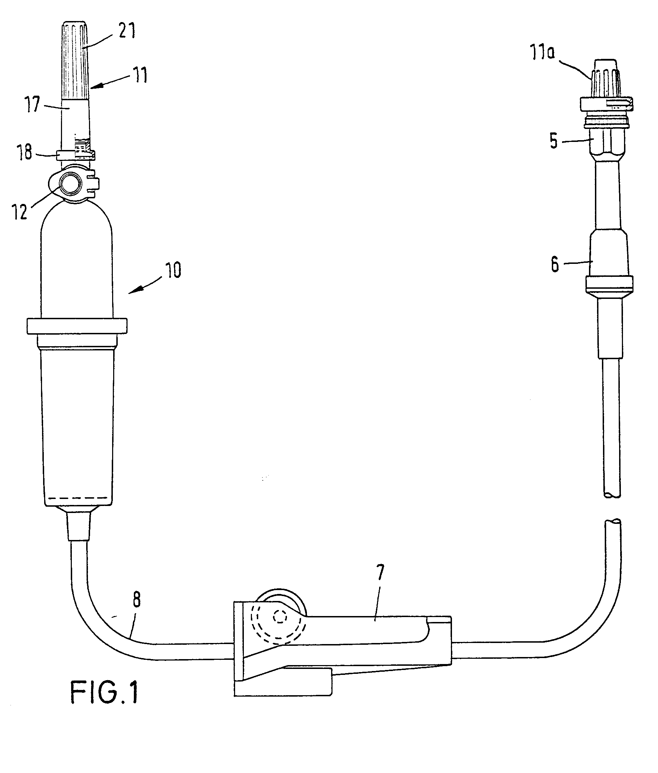

Das in Fig. 1 dargestellte medizinische Übertragungsgerät besteht aus einer Tropfkammer 10, einem Schlauch 8, einer Rollenklemme 7, einem Injektionszwischenstück 6 und einem Verbindungsstück 5. An den Konnektierungsstellen an Tropfkammer 10 und Verbindungsstück 5 ist das medizinische Gerät jeweils mit Schutzkappen 11,11 a ausgestattet.

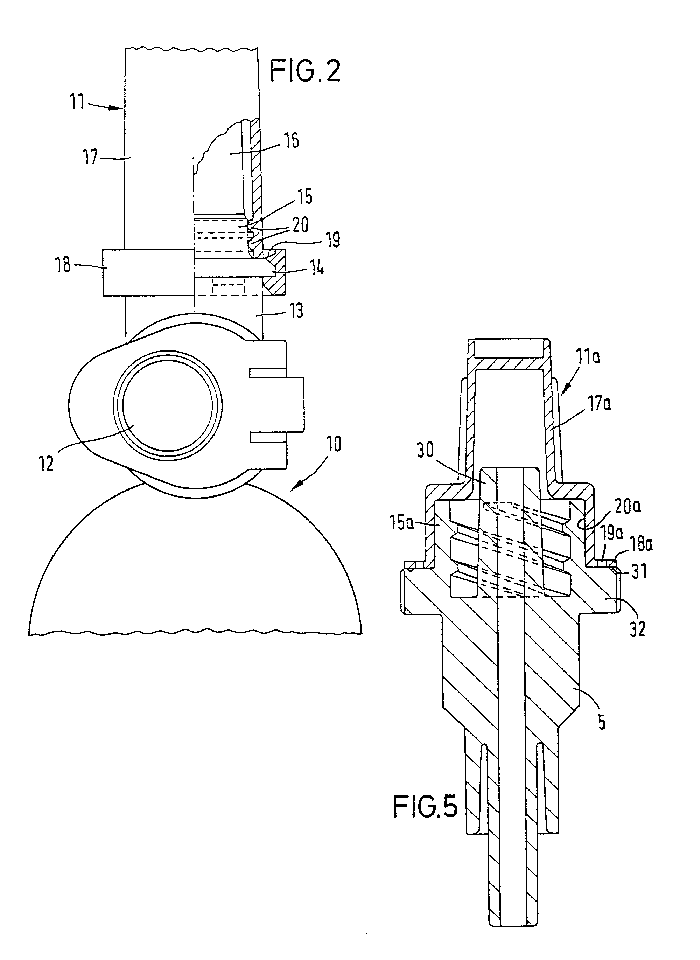

Gemäß Fig. 2 weist die Tropfkammer 10 einen Sockel 13 mit einem flanschartigen Kragen 14 auf. Von dem Kragen 14 steht axial ein zylindrischer Ansatz 15 ab, aus dem der Schaft 16, bei dem es sich hier um den Einstechdorn handelt, aufragt.

Die Schutzkappe 11 besteht aus dem länglichen Kappenkörper 17 und dem damit einstückig verbundenen Halteteil 18. Der Kappenkörper 17 hat die Form eines langgestreckten Köchers, der nur an einem Ende offen ist. An diesem offenen Ende befindet sich das Halteteil 18. Das Halteteil 18 umgibt das untere Ende des Kappenkörpers 17 ringförmig und es weist nach innen vorstehende Ansätze auf, welche durch Abreißteile 19 mit dem Rand des Kappenkörpers 17 verbunden sind. Diese Abreißteile 19 sind umfangsmäßig verteilt angeordnete Stege, zwischen denen sich Schlitze befinden.

Das Halteteil 18 ist mit dem Kragen 14 der Tropfkammer 10 derart verbunden, daß es nicht zerstörungsfrei entfernt werden kann. Das Halteteil 18 umschließt den Kragen 14 von entgegengesetzten Enden her und erstreckt sich über den gesamten Umfang des Kragens.

Am unteren Ende des Kappenkörpers 17 ist im Inneren des Kappenkörpers ein aus ringförmigen Wülsten bestehender Sitz 20 vorgesehen, der den Ansatz 15 der Tropfkammer 10 keimdicht umschließt. Auf diese Weise wird das Innere des Kappenkörpers 17 gegen Kontamination geschützt.

Wie Fig. 1 zeigt, ist der Kappenkörper an seiner Außenseite mit Griffteilen 21 versehen, die ein leichtes Abscheren des Kappenkörpers 17 vom Halteteil 18 ermöglichen.

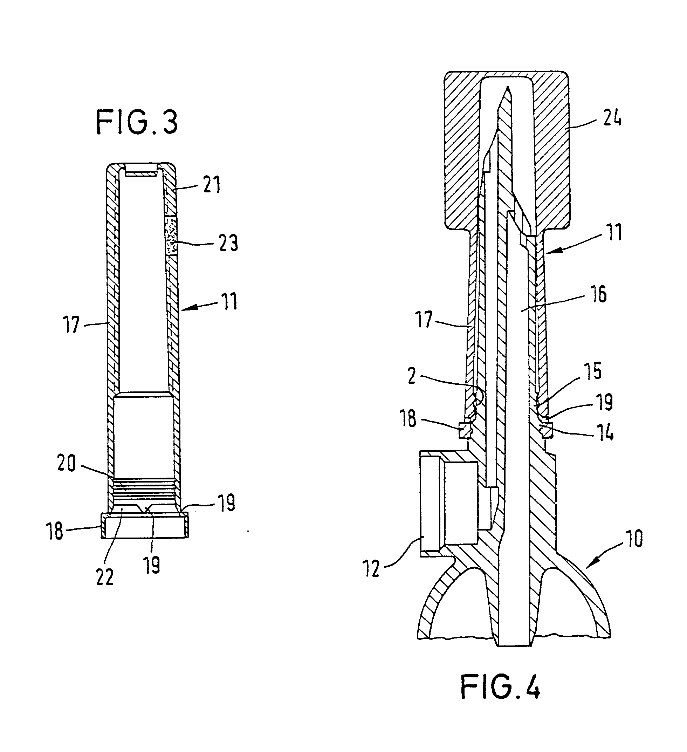

Fig. 3 zeigt eine andere Ausführungsform der Schutzkappe 11, z.B. für eine Klebeverbindung des Halteteils mit dem Sitz am medizinischen Gerät, bei der der Kappenkörper 17 ebenfalls durch Abreißteile 19 mit dem Halteteil 18 verbunden ist. Zwischen den Abreißteilen 19 befinden sich Schlitze 22. Oberhalb der Schlitze ist im Kappenkörper 17 ein Sitz 20 aus mehreren hintereinander angeordneten, ringförmigen Wülsten vorgesehen. An der Außenseite des Kappenkörpers befinden sich Griffteile 21 in Form von Stegen. Bei der Anbringung der Schutzkappe an dem medizinischen Gerät wird das unverformte Halteteil 18 über den Kragen des Gerätes geschoben und verklebt.

Die Schutzkappe nach Fig. 3 weist an ihrer Wandung einen gasdurchlässigen Sterilfilter 23 auf, um eine Gas- oder Dampfsterilisation im Inneren der Schutzkappe durchführen zu können, wenn die Schutzkappe auf dem Gerät montiert ist.

Fig. 4 zeigt eine weitere Ausführungsform des Gerätes mit dem von der Schutzkappe 11 bedeckten Schaft 16, der hier wiederum der Einstechdorn eines Übertragungsgerätes ist. Die Schutzkappe besteht aus dem köcherförmigen Kappenkörper, der an seinem einzigen offenen Ende über Abreißteile 19 mit dem ringförmigen Halteteil 18 verbunden ist. Der Kappenkörper 17 ist mit seitlich abstehenden Flügeln 24 versehen, die als Griffteile das Abdrehen der Abreißteile 19 erleichtern.

Gemäß Fig. 5 ist das Anschlußstück 5, das nach Art eines Luer-Lock-Konnektors ausgebildet ist, mit einer Verschlußkappe 11 versehen, die auf die Fassungshülse 15a des Verbindungsstücks 5 aufgesetzt ist und den konischen Schaft 30 gegen die Umgebung keimdicht abschirmt. Der Kappenkörper 17a weist an seiner Innenseite einen Sitz 20a auf, der die zylindrische Umfangswand der Fassungshülse 15a abdichtend umgibt. Am rückwärtigen Ende des Kappenkörpers 17a befindet sich ein nach außen abstehender Flansch, der das Halteteil 18a bildet, welches durch Schweißung 31 am Umfangsflansch 32 des Anschlußstückes 15a dauerhaft befestigt ist. An dem Flansch 18 sind innerhalb des von der Schweißlinie 31 umschlossenen Bereichs Abreißteile 19a vorgesehen, die umfangsmäßig verteilt angeordnet und durch Schlitze voneinander getrennt sind. Durch Drehen des Kappenkörpers 17a werden diese Abreißteile abgeschert und der Kappenkörper kann von dem Verbindungsstück 5 abgenommen werden, während das Halteteil 18a am Verbindungsstück verbleibt.

## Patentansprüche

1. Medizinisches Übertragungsgerät mit einem Schlauch (8) an dessen einem Ende sich eine Tropfkammer (10) mit einem Einstechdorn (16) befindet und dessen anderes Ende mit einem Anschlußstück (5) versehen ist, wobei auf einem Ansatz (15,15a) des Einstechdornes (16) und/oder des Anschlußstücks (5) eine Schutzkappe (11,11 a) sitzt, dadurch gekennzeichnet, daß die Schutzkappe (11,11 a) einen abnehmbaren Kappenkörper (17,17a) aufweist, der mindestens ein Abreißteil (19,19a) mit einem nicht zerstörungsfrei lösbaren, an dem Einstechdorn bzw. der Schutzkappe angebrachten Halteteil.

2. Medizinisches Übertragungsgerät nach Anspruch 1, dadurch gekennzeichnet, daß der Kappenkörper (17) einen den Ansatz (15,15a) des Einstechdornes (16) bzw. der Schutzkappe (11,11 a) keimdicht umschließenden Sitz (20) aufweist.

3. Medizinisches Übertragungsgerät nach Anspruch 1 oder 2, dadurch gekennzeichnet, daß mehrere Abreißteile (19) in Form von umfangsmäßig beabstandeten Stegen vorgesehen sind, zwischen denen sich Schlitze (22) befinden.

4. Medizinisches Übertragungsgerät nach einem der Ansprüche 1 bis 3, dadurch gekennzeichnet, daß der Kappenkörper (17) und das Halteteil (18) aus einem einstückigen Kunststoffteil bestehen.

5. Medizinisches Übertragungsgerät nach Anspruch 4, dadurch gekennzeichnet, daß die Medizinisches Übertragungsgerät aus Kunststoff besteht.

6. Medizinisches Übertragungsgerät nach einem der Ansprüche 1 bis 5, dadurch gekennzeichnet, daß der Kunststoff für die Strahlensterilisation geeignet ist.

7. Medizinisches Übertragungsgerät nach einem der Ansprüche 1 bis 5, dadurch gekennzeichnet, daß der Kappenkörper (17) einen gasdurchlässigen Sterilfilter (23) aufweist, um eine Gassterilisation zu ermöglichen.
